# EUROPÄISCHE PATENTSCHRIFT

(11) **EP 0 959 783 B1**
(45) Veröffentlichungstag und Bekanntmachung des Hinweises auf die Patenterteilung: **29.12.2004**
(21) Anmeldenummer: 98904118.1
(22) Anmeldetag: 22.01.1998
(51) Int. Cl.: A61B 18/22

(54) **VORRICHTUNG ZUR LASERABLATION VON GEWEBE**
TISSUE LASER ABLATION DEVICE
DISPOSITIF D'ABLATION PAR LASER DE TISSUS

(30) Priorität: 29.01.1997 DE 19703208
(43) Veröffentlichungstag der Anmeldung: 01.12.1999
(73) Patentinhaber: Carl Baasel Lasertechnik GmbH, D-82319 Starnberg (DE)
(72) Erfinder: BAUMGARTNER, R., D-85345 Freising (DE); HOFSTETTER, A., D-82008 Unterhaching (DE); MUSCHTER, R., D-82319 Starnberg (DE); PERLMUTTER, A., P., New York, NY 10128-7834 (US); SROKA, R., D-80935 München (DE); FALKENSTEIN, W., D-82319 Starnberg (DE)
(74) Vertreter: Feldkamp, Rainer, Dipl.-Ing.
(86) Internationale Anmeldenummer: PCT/EP1998/000335
(87) Internationale Veröffentlichungsnummer: WO 1998/032381

(56) Entgegenhaltungen:
- EP-A- 0 125 897
- WO-A-93/06888
- US-A- 5 196 005
- US-A- 5 246 436
- US-A- 5 303 324
- US-A- 5 486 171

## Beschreibung

Die Erfindung bezieht sich auf eine Vorrichtung der im Oberbegriff des Anspruchs 1 genannten Art.

Dieses Verfahren bzw. die Vorrichtung ist insbesondere zur Laserablation der Prostata vorgesehen, eignet sich jedoch in gleicher Weise zur Therapie benigner und maligner Wucherungen, wie z.B. der der Niere, der Leber oder des Uterus.

Die benigne Prostatahyperplasie (BPH; gutartige Vergrößerung der Prostata) betrifft bereits ca. 50% der 50jährigen Männer und nimmt in ihrer Inzidenz bis zu 95% der über 80jährigen Männer zu. Das Beschwerdebild ist uneinheitlich und unterliegt großen interindividuellen Schwankungen; neben einer Abschwächung des Harnstrahls und dem verzögerten Beginn des Wasserlassens bis zur völligen Harnverhaltung steht der häufige tägliche und vor allem nächtliche Harndrang im Vordergrund.

Die drei Komplexe Vergrößerung-Obstruktion-Symptome, die die Erkrankung charakterisieren, zeigen keine Korrelation.

Aus diesem Grund gibt es eine Vielzahl von therapeutischen Optionen. Selbstverständlich steht die Linderung der Symptome (denn unter diesen leidet der Patient) an erster Stelle, zur Behandlung der Ursache ist aus medizinischer Sicht jedoch auch die Beseitigung der Obstruktion und die Verkleinerung der Prostata erforderlich oder zumindest wünschenswert. Somit gilt nach Scheitern der oftmals über Jahre durchgeführten medikamentösen Therapie nach wie vor die operative Therapie als "Gold"-Standard. Diese kann offen operativ erfolgen (Ausschälung der drüsigen Geschwulst), die Regel ist allerdings die transurethrale Resektion der Prostata (TURP; "Hobelung"). Hierbei wird das hyperplastische Gewebe mit einer Hochfrequenzschneideschlinge, die in einen Blasenspiegel integriert ist, durch die Harnröhre stückchenweise vollständig entfernt.

Wegen der Risiken, Komplikationen und unvermeidlichen Nebenwirkungen dieses Verfahrens wurden in der letzten Jahren zahlreiche "minimal-invasive" Methoden populär, die meist eine thermische Zerstörung der BPH anstreben. Verschiedene Energiequellen (u.a. Mikrowellen, radiofrequente Wellen, Nd:YAG- und Diodenlaser mit Wellenlängen des nahen Infrarotbereiches, fokussierter Ultraschall, Hochfrequenzstrom, Mantelheizleiter) und Applikationsmodi (transrektal, transurethral, interstitiell) wurden dazu vorgestellt. Für die thermische Zerstörung kann entweder eine Koagulation (z.B. TUMT = transurethrale Mikrowellen-Thermotherapie, VLAP = visuelle Laserablation der Prostata, ILK = interstitielle Laserkoagulation, TUNA = transurethrale Nadelablation, HIFU = fokussierter Ultraschall hoher Intensität) oder eine Vaporisation (z.B. TUEP = transurethrale Evaporisation der Prostata, Kontaktlaservaporisation, TVP = transurethrale Elektrovaporisation der Prostata) angestrebt werden. Die Vielzahl der konkurrierenden Konzepte weist darauf hin, daß sie nicht ohne Nachteile sind.

Die Koagulation der Prostata erfolgt entweder durch transurethrale oder durch interstitielle Energieapplikation. Bei der transurethralen Applikation befindet sich in der Regel ein die Energie gerichtet (z.B. Laserlichtleiter mit Strahlumlenkung) oder ungerichtet (z.B. Mikrowellenantenne) abgebender Applikator in der prostatischen Harnröhre. Da diese nur bedingt gekühlt werden kann, wird sie in der Regel zerstört. Das thermisch zerstörte (nekrotische) Gewebe verbleibt in situ. Dieses löst sich innerhalb der folgenden Tage bis Wochen bzw. Monaten ab und wird mit dem Harn ausgeschieden. Darunter kommt es zu einer normalen Wundheilung. Der Hauptvorteil ist die fehlende Blutung, der Hauptnachteil ist die Notwendigkeit der posttherapeutischen Harnableitung über einen Katheter, da es durch die mit der Wärmeapplikation einhergehenden ödematösen Schwellung und die Verhärtung des in situ verbleibenden koagulierten Gewebes zu einer Behinderung des Wasserlassens, oft sogar zur Harnverhaltung kommt, die bis zur ausreichenden Gewebeablösung bestehen bleibt. Relative Nachteile gegenüber der in der Folge beschriebenen interstitiellen Koagulation sind die Gewebeabstoßung, die manchmal irritative Symptome (z.B. schmerzhafter oder imperativer Harndrang) verursachen können und das größere Risiko der Verletzung des inneren und äußeren Schließmuskels.

Die interstitielle Koagulation besitzt diese Nachteile nicht. Hier wird ein Applikator (z.B. Laserlichtleiter, Radiofrequenzantenne oder Mantelheizleiter) direkt in das Gewebe eingestochen (durch die Harnröhre oder die Haut des Perineums) oder die Energie so abgegeben (z.B. perkutan oder transrektal mittels in die Prostata fokussiertem Ultraschall oder transurethral mittels einer ausreichend gekühlten in der Urethra gelegenen Antenne), daß die Koagulation innerhalb der hyperplastischen Prostatalappen erfolgt, ohne umliegende Organe oder die Urethra zu beeinträchtigen. Auch diese Techniken sind praktisch ohne Blutungsrisiko und vermeiden die weiteren Risiken der operativen Verfahren, besitzen aber ebenfalls den Nachteil der posttherapeutisch notwendigen Harnableitung über einen Katheter bis der Abbau des nekrotischen Gewebes innerhalb der Prostata zu einer ausreichenden Schrumpfung geführt hat (Zeitdauer mehrere Wochen bis Monate).

Bei der Vaporisation (mit Laser oder Hochfrequenzstrom) wird versucht, diesen Nachteil durch sofortiges Abtragen des thermisch zerstörten Gewebes zu vermeiden. Die Nachteile der Vaporisation bestehen in erster Linie in dem hohen Zeitbedarf für einen ausreichenden Volumenabtrag, jedoch auch in dem höheren Risiko, daß TURP-typische Komplikationen, wie z.B. eine Blutung, auftreten. Wie bei der transurethralen Koagulation wird die Vaporisation transurethral unter Zerstörung der Urethra durchgeführt und geht mit dem Risiko der Zerstörung des inneren und äußeren Schließmuskels einher.

Mit der Vaporisation ist der Trend zu einer weitgehenden Imitation der transurethralen Resektion der Prostata zu erkennen. Man möchte zwar die Risiken, Komplikationen und Nebenwirkungen der TUR, aber auch den Hauptnachteil der thermischen Koagulation, die langdauernde Harnableitung mittels Katheter, vermeiden.

Konsequent wurde nach Techniken gesucht, die ggf. auch den Nachteil der Vaporisation, den hohen Zeitbedarf, beseitigen. Hieraus entstanden in der jüngeren Vergangenheit neue Resektionstechniken, die im wesentlichen eine Modifikation der herkömmlichen TURP (z.B. mit neuartigen Hochfrequenzgeneratoren oder Schneideschlingen mit größerer Masse) darstellen. In diesem Zusammenhang wurden auch Verfahren entwickelt, bei denen Laserlichtleiter im Kontakt zur transurethralen Resektion der Prostata benutzt werden, u.a. der Holmium:YAG-Laser für die sogenannte Holmiumlaser-Resektion der Prostata (HoLRP). Nachteilig sind hierbei die erforderliche hohe Laserleistung (ca. 60 W) und die damit verbundene Gefahr bei Fehlapplikationen sowie die hohen Kosten eines derartigen Gerätes.

Die WO 93/06888 beschreibt ein Lasergerät, das zur Behandlung von Wurzelkanälen des Zahnes geeignet ist. Die Abstrahlung von Laserlicht erfolgt hierbei ringförmig, eine apikale Abstrahlung soll vermieden werden. Um dies zu erreichen, werden entweder Silberspiegel eingesetzt oder wird der äußere Teil der Faseroptik speziell ausgeformt.

Nachteil dieser Vorrichtung ist, daß zur Strahlumlenkung zusätzlich reflektive oder refraktive Materialien benötigt werden und daß ein interstitieller Einsatz, wie er beispielsweise bei der Behandlung der BPH erforderlich ist, nicht möglich ist.

Die US-Patentschrift 5 486 171 offenbart ein Lasergerät, daß zu einer monodirektionalen Ablenkung eines Laserstrahls in der Lage ist. Die Ablenkung erfolgt hier ohne zusätzliche reflektierende Materialien, sondern durch die Eigenschaften des Quarzmaterials der Faseroptik, der die Faseroptik umgebenden transparenten Hülse sowie des den Raum zwischen Faseroptik und Hülse füllenden Mediums.

Nachteilig ist hierbei, daß diese Vorrichtung nicht zu einem interstitiellen Einsatz brauchbar ist und daß keine großvolumigen Gewebszerstörungen möglich sind.

Die US-Patentschrift 4 878 725 beschreibt ein Lasergerät, bei dem die Lichtstrahlung unter einem vorgegebenen Winkel zu der zur Eingangs-Endfläche des Lichtleiters senkrechten Ebene derart eingekoppelt wird, daß die Lichtstrahlung das entfernte Ende des Lichtleiters innerhalb eines konischen, nach vorne gerichteten Raumes verläßt. Um dieses freie Ende herum kann eine lichtdurchlässige Kappe angeordnet sein, die einen luftgefüllten Hohlraum mit dem Ende des Lichtleiters umschließt. Hierdurch erfolgt eine weitere Strahlumlenkung eines Teils der konischen Lichtstrahlung, doch wird diese Lichtstrahlung über ein großes Volumen verteilt, so daß die Energiedichte der Lichtstrahlung relativ gering ist.

Aus der Veröffentlichung von Johnson et al: "Interstitial Laser Prostatectomy", Lasers in Surgery and Medicine 14(1994), No.4, Seiten 299-305 ist ein Ver fahren bzw. eine Vorrichtung nach dem Oberbegriff des Anspruchs 1 bekannt. Dabei wird jedoch eine optische Faser mit einem Durchmesser von 600 µm eingesetzt, deren Endabschnitt über eine Strecke von 20 mm derart geschliffen ist, daß sich in diesem Bereich eine radiale, insgesamt also zylinderförmige Auskopplung der Lichtenergie ergibt. Die optische Faser ist mit einem transparenten Schutzüberzug versehen, der einen Durchmesser von 1,8 mm aufweist.

Aus der US-Patentschrift 5 354 293 ist eine Vorrichtung zur Bestrahlung der Innenwände eines Hohlraumes vorgesehen, d.h. es ist keine interstitielle Einführung im Sinn der Erfindung vorgesehen. Die Vorrichtung soll eine möglichst gleichmäßige Licht- bzw. Energie-Verteilung über die gesamten Innenwände des Hohlraums ermöglichen. Hierzu wirkt die konische Spitze der optischen Faser mit dem aufpumpbaren sie umgebenden Bereich des Katheders derart zusammen, daß sich die erwünschte kugel- oder elipsoidförmige Energieabstrahlung ergibt.

Die US-Patentschrift 5 312 392 betrifft ebenfalls ein Verfahren zur Laserablation von Gewebe, wobei auch dort vorgesehen ist, daß der Lichtleiter die Energie in gestreuter bzw. diffuser Form abgibt.

Der Erfindung liegt die Aufgabe zugrunde, ein einfach und schnell durchzuführendes Verfahrens bzw. eine einfach aufgebaute Vorrichtung zur Laserablation von Gewebe der eingangs genannten Art zu schaffen, das bzw. die einen unmittelbaren Gewebeabtrag mit möglichst geringen Komplikationsmöglichkeiten bei vergleichsweise geringen Laserleistungen und Gerätekosten erreicht.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Durch die erfindungsgemäße Ausgestaltung der Vorrichtung ist es möglich, die Nachteile bekannter Resektionstechniken (z.B. Blutungsrisiko), der Vaporisation des Gewebes und der Koagulation des Gewebes (z.B. lange postoperative Katheterzeit im Fall von Prostatagewebe) zu beseitigen. Bei der erfindungsgemäßen Vorrichtung wird eine interstitielle Ablation des Gewebes von definierter Lokalisation und definiertem Ausmaß mit angrenzender, ausreichend großer Koagulation erreicht, die zuverlässig Blutungen verhindert.

Bei der Vorrichtung wird die Strahlung z.B. eines gepulsten Ho:YAG-Lasers der Wellenlänge 2120 nm ("mittlerer" Infrarotbereich) über einen interstitiellen Applikator mit einem speziellen Lichtleiter mit zirkulärer Abstrahlung interstitiell (d.h. innerhalb des Gewebes) appliziert. Es ist auch möglich, die gewünschten Gewebeeffekte mit anderen Lichtquellen (Lasern) geeigneter Parameter (Wellenlänge 1000 bis 3500 nm, Impulsdauer 50 bis 1000 µs, Impulsenergie 1 bis 6 J) zu erreichen, wenn die Schwelle für die Gewebeablation überschritten wird. Beispiele derartiger Laser sind Tm:YAG-, Nd:YAG- und Er:YAG-Laser, wobei diese Aufzählung nicht abschließend ist.

Durch die erfindungsgemäße Konzentration der Laserenergie auf einen ring- bzw. scheibenförmigen Bereich kann die Ablationsschwelle sowohl hinsichtlich der Energie- als auch Leistungsdichte mit kommerziell erhältlichen Lasern mittlerer Leistungsklasse überschritten werden.

Der Lichtleiter des erfindungsgemäßen Applikators weist zu diesem Zweck an seinem freien, in das Gewebe einzustechenden Bereich eine Strahlumlenkeinrichtung in Form einer konisch ausgebildeten Spitze auf, wobei dieses freie Ende mit der konischen Spitze in einer für das Laserlicht durchlässigen Hülse angeordnet ist, die das freie Ende mit Abstand umgibt, so daß zwischen dem freien Ende des Lichtleiters und dem Innenumfang der Hülse ein Zwischenraum gebildet wird, der mit Luft oder einem anderen Gas oder Medium gefüllt sein kann, das derartige Brechungseigenschaften an der Grenzfläche zwischen der konischen Spitze und dem Medium ergibt, daß das Laserlicht unter einem Winkel im Bereich von 40 bis 140°, vorzugsweise etwa 90°, zur Lichtleiterachse ausgekoppelt wird. Auf diese Weise ergibt sich ein scheiben- oder ringförmiger Bestrahlungsbereich, der im Querschnitt auf jeder Seite der Achse des Lichtleiters konisch von dem Lichtleiter aus erweitert ist und in dem nahezu die gesamte Laserenergie konzentriert ist.

Die Hülse kann an dem freien Ende des Lichtwellenleiters mit Hilfe geeigneter Mittel, beispielsweise durch Verkleben, befestigt sein, wobei diese Verklebung in einem ausreichenden Abstand rückwärts der Spitze erfolgt.

Ausführungsbeispiele der Erfindung werden im folgenden anhand der Zeichnungen noch näher erläutert.

In der Zeichnung zeigen:
Fig. 1 eine vereinfachte schematische Darstellung einer Ausführungsform des Applikators mit einer das freie Ende eines Lichtleiters umgebenden Hülse,
Fig. 2 eine Darstellung der Intensität der Laserenergie über einem Winkel zur Längsachse des Lichtleiters,
Fig. 3 ein Beispiel für die Anwendung des in Fig. 1 gezeigten interstitiellen Applikators,
Fig. 4 eine Darstellung der Wirkung einer mehrfachen Anwendung des interstitiellen Applikators nach Fig. 1,
Fig. 5 eine den Fig. 3 und 4 entsprechende Darstellung nach Eröffnung des Ablationsbereichs zur Urethra hin.

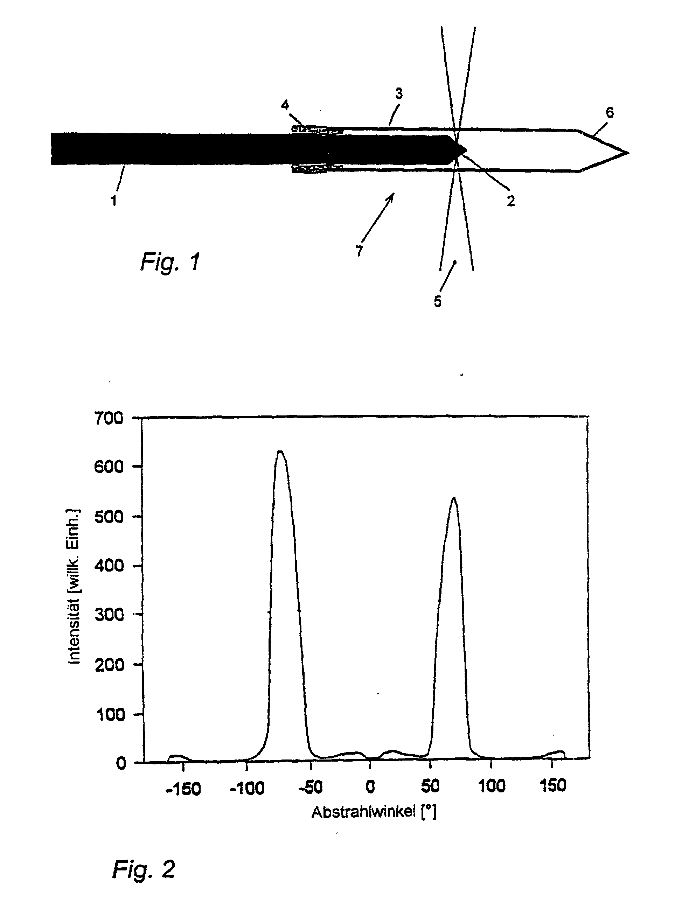

In Fig. 1 ist eine Ausführungsform des erfindungsgemäßen interstitiellen Applikators gezeigt, der insgesamt mit 7 bezeichnet ist und das freie Ende eines am anderen Ende mit einer Laserlichtquelle 10 (Fig. 2) verbundenen Lichtleiters 1 einschließt. Dieses freie Ende des Lichtleiters 1 weist eine Strahlumlenkeinrichtung in Form einer konischen Spitze 2 auf, und dieses freie Ende ist von einer für das Laserlicht durchlässigen Hülse 3 umgeben, die zur Vereinfachung der interstitiellen Anwendung ebenfalls mit einer Spitze 6 versehen ist, die ein Einstechen in Gewebe ermöglicht.

Die Hülse 3 ist an der mit 4 bezeichneten Stelle durch Verkleben oder durch andere Einrichtungen mit der Ummantelung des Lichtleiters 1 verbunden und an diesem Lichtleiter festgelegt. Die Hülse 3 umgibt das freie Ende des Lichtleiters 1 und dessen Spitze 2 mit einem entsprechenden Abstand, so daß zwischen der Hülse 3 und dem freien Ende des Lichtwellenleiters 1 ein Hohlraum gebildet wird, der mit einem Gas oder einem anderen Medium gefüllt ist, das eine Brechung und Beugung der entlang der Achse des Lichtleiters 1 verlaufenden Strahlen an den Mantelflächen der konischen Spitze 2 derart ermöglicht, daß sich eine radiale Auskopplung dieses Lichtes in den bei 5 schematisch angedeuteten Ringraum ergibt. Auf diese Weise kann durch entsprechende Bemessung der Spitze 2, des Materials der Hülse 3 und des den Zwischenraum zwischen der Hülse 3 und dem freien Ende des Lichtleiters 1 füllenden Mediums eine ring- oder scheibenförmige Auskopplung des Lichtes erreicht werden, wie dies in Figur 2 gezeigt ist. Das ausgekoppelte Laserlicht ist damit in einem Ring oder einer Scheibe konzentriert, deren Höhe sich ausgehend von der Achse des Lichtleiters konisch vergrößert, wobei die Hauptenergie in einem Winkel zwischen etwa ± 50° und ± 90° zur Längsachse des Lichtleiters konzentriert sein kann, wie dies aus Figur 2 zu erkennen ist. Bevorzugt wird hierbei ein Winkel von ± 90° mit einem Divergenzwinkel im Bereich von 10° bis 30°

Die von der Laserlichtquelle 10 in dem Lichtleiter 1 eingespeiste Energie ist so hoch, daß sich in diesem ringförmigen Bereich 5 eine für eine Ablation in dem entsprechenden Gewebe ausreichende Energie- bzw. Leistungsdichte ergibt.

Der Spitzenwinkel der Spitze 2 beträgt beispielsweise 85°, wobei sich bei Füllung des Zwischenraumes zwischen der Hülse 3 und dem freien Ende des Lichtleiters 1 mit Luft beispielsweise ein Ablenkwinkel aus der Achse des Lichtleiters von 85° ergibt, und die Divergenz der Laserstrahlung in dem Ring 5 etwa 20° beträgt. Hierbei hat der Ring in einer Entfernung von 3 bis 5 mm von der Achse des Lichtleiters etwa eine Höhe (gemessen längs der Lichtleiterachse) von 1,5 bis 2 mm.

Die verwendete Laserlichtquelle ist vorzugsweise ein Ho:YAG-Laser mit einer Wellenlänge von 2120 nm, d.h. im mittleren Infrarot-Bereich. Bei Verwendung dieses Lasers ist es wichtig, daß sowohl der Lichtleiter 1 als auch die Hülse 3 aus einem Glasmaterial mit niedrigem OH-Gehalt besteht, um die optische Dämpfung zu verringern.

Die Energie dieser Laserlichtquelle beträgt beispielsweise 2 J pro Impuls mit einer Impulsdauer von 400 µs.

Bei praktisch ausgeführten Versuchen wurden hierbei Lichtleiter mit einem Kerndurchmesser im Bereich von 0,2 bis 0,8 mm verwendet, was z.B. bei einem Kerndurchmesser von 0,4 mm zu einer Bestrahlung in dem ringförmigen Bereich von 4,2 J/cm² führte.

Wie tierexperimentell gezeigt werden konnte, entsteht bei einer z.B. einminütigen Bestrahlung mit geeigneten Laserparametern (2J Einzelimpulsenergie, 5 Hz) im Bereich des durch das Ende des Lichtleiters 1 und die Hülse 3 gebildeten Applikators eine annähernd kugelförmige großvolumige Kavität, die von einer schmalen Karbonisation sowie mehrere mm breiten Koagulation umgeben ist. Blutungen wurden weder in der Zone der thermischen Schädigung, noch an anderer Stelle beobachtet. Alle umliegenden Organe und Gewebe wurden nicht geschädigt oder beeinträchtigt.

In der Folgezeit kam es innerhalb von 3 Wochen zur vollständigen Abheilung der thermischen Schädigung unter Zurückbleiben eines großen Defektes mit Ausbildung einer großen zentralen Kavität. Diese war nach 6 Wochen unverändert nachweisbar.

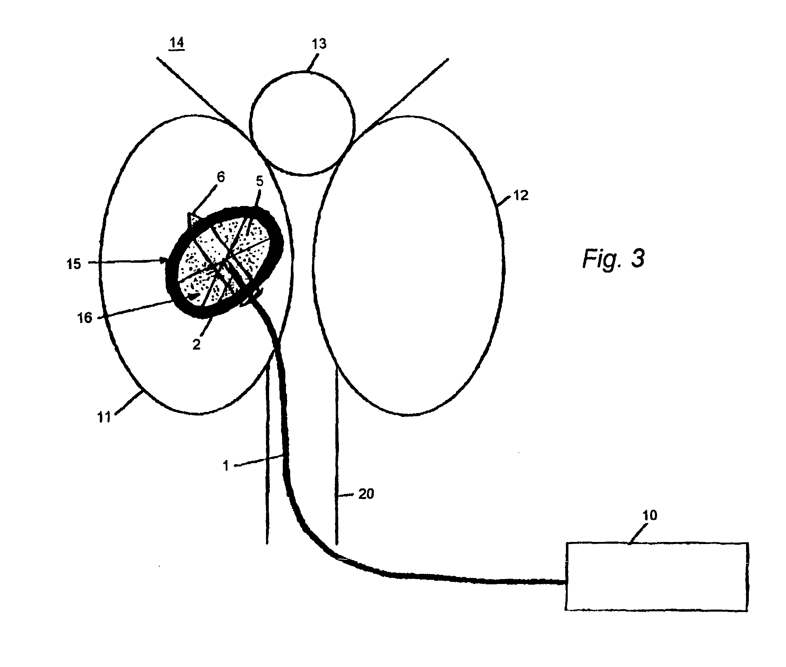

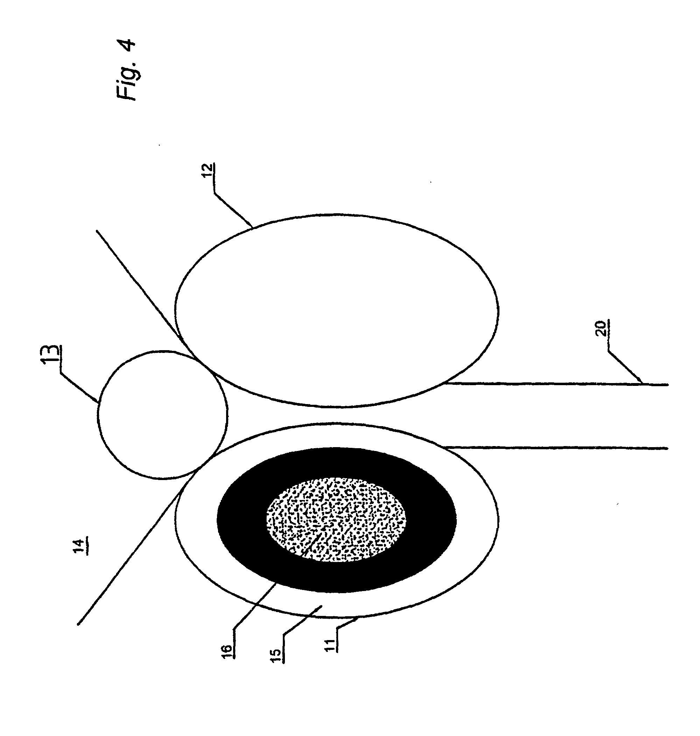

Für die klinische Anwendung wird der Applikator im Fall der Behandlung der Prostata z.B. transurethral über ein herkömmliches Zystoskop oder perkutan über eine Punktionshülse in die Prostata, beispielsweise in den Prostataseitenlappen 11 gemäß Figuren 3 bis 5, eingebracht, wobei Figur 3 den Zustand nach einer ersten Punktion und Figur 4 den abschließenden Zustand nach mehreren Punktionen zeigt. Die Einbringung (Punktion) erfolgt wiederholt in jeweils andere benachbarte Bereiche des zu behandelnden Gewebes, so daß das gesamte zu zerstörende Gewebe bestrahlt wird. Jede einzelne Punktion wird nach den Erfordernissen der individuellen Behandlung durchgeführt, beispielsweise apikal parallel zur Urethra in das Zentrum des Seitenlappens, oder mehr nach ventral oder dorsal gerichtet u.s.w.

Als Ergebnis entsteht eine großvolumige thermische Schädigung in Form eines von einer Koagulationszone 15 umgebenen Ablationsbereiches 16 innerhalb der Prostata. Der Zeitbedarf beträgt nur wenige Minuten. Das Auftreten von Blutungen oder anderen TURP-typischen Operationskomplikationen ist nicht zu erwarten. In Analogie zu den Techniken der interstitiellen Koagulation und aufgrund der experimentellen Erfahrungen wird die thermische Schädigung innerhalb einiger Wochen mit dem erwünschten großen Defekt abheilen und sich eine große Kavität im Bereich der prostatischen Harnröhre ausbilden. Anders als bei der Koagulation ist jedoch bereits unmittelbar nach Beendigung der Behandlung durch Verdampfen des Gewebes im Ablationsbereich eine Kavität entstanden, so daß es trotz thermischem Ödem und peripherer Verhärtung des Gewebes in der Summe zu einer sofortigen Abnahme der Obstruktion kommt, da das obstruierende Gewebe sich in Richtung auf die Kavität zurückzieht.

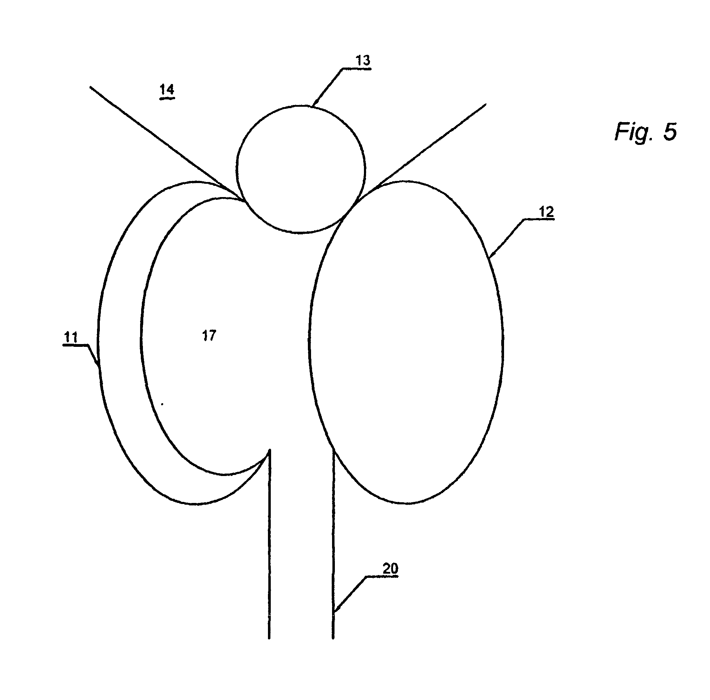

Alternativ kann z.B. mit dem Laser oder einem herkömmlichen Resektionsinstrument die Urethra und das periurethrale Gewebe soweit abgetragen werden, bis die zentrale Kavität eröffnet wird, wie dies in Figur 5 gezeigt ist. Hierdurch wird das Lumen der prostatischen Harnröhre unmittelbar erheblich erweitert. Die zu erwartenden Blutungen bei dieser Resektion sind allenfalls minimal und der Zeitbedarf ist gering.

Entsprechende Anwendungen des Systems und des Verfahrens sind auch an den anderen Organen, z.B. der Niere zur Behandlung lokalisierter gut- oder bösartiger Tumore möglich.

## Patentansprüche

1. Vorrichtung zur Laserablation von Gewebe, mit einer Laserlichtquelle (10), deren Ausgangsleistung in ein erstes Ende eines Lichtleiters (1) eingespeist wird, wobei die am freien Ende des Lichtleiters (1) austretende Lichtenergie auf das Gewebe (11) gerichtet wird, um die Ablation des Gewebes hervorzurufen, wobei das freie Ende des Lichtleiters mit Abstand von einer interstitiell in das Gewebe (11) einbringbaren im wesentlichen zylindrischen Hülse (3) umgeben ist, die aus für das Laserlicht im wesentlichen durchlässigem Material besteht, und wobei an dem freien Ende des Lichtleiters (1) Strahlumlenkeinrichtungen (2,3) zur Umlenkung des aus dem Lichtleiter austretenden Lichtstrahls angeordnet sind,
**dadurch gekennzeichnet,**
**daß** das freie Ende des Lichtleiters durch eine konische Spitze (2) mit einem Spitzenwinkel gebildet ist, daß die Strahlumlenkeinrichtungen (2,3) durch die den Spitzenwinkel aufweisende konische Spitze (2), die Hülse (3) und das den Zwischenraum zwischen der Spitze (2) und der Hülse (3) füllende Medium gebildet ist, und daß die Strahlenumlenkeinrichtung (2,3) eine Auskopplung der Lichtenergie in einem scheiben- oder ringförmigen Raum (5) unter einem Winkel zur Längsachse ergibt.

2. Vorrichtung nach Anspruch 1,
**dadurch gekennzeichnet, daß** der Winkel zur Längsachse im Bereich von etwa 40 bis 140° liegt.

3. Vorrichtung nach Anspruch 1 oder 2,
**dadurch gekennzeichnet, daß** der Winkel der Winkel zur Längsachse etwa 90° beträgt.

4. Vorrichtung nach einem der Ansprüche 1 bis 3,
**dadurch gekennzeichnet, daß** die Hülse an ihrem freien Ende eine Spitze (6) aufweist, die das Einbringen in das Gewebe erleichtert.

5. Vorrichtung nach einem der Ansprüche 1 bis 4,
**dadurch gekennzeichnet, daß** der Spitzenwinkel der Spitze (2) im Bereich von 85° liegt.

6. Vorrichtung nach einem der Ansprüche 1 bis 5,
**dadurch gekennzeichnet, daß** die in den ring- oder scheibenförmigen Raum (5) abgegebene Strahlung einen Divergenzwinkel im Bereich von 20° aufweist.

7. Vorrichtung nach einem der Ansprüche 1 bis 16,
**dadurch gekennzeichnet, daß** der Außendurchmesser der Hülse im Bereich von 0,5 bis 3 mm, vorzugsweise bei 2 mm liegt.

8. Vorrichtung nach einem der Ansprüche 1 bis 7,
**dadurch gekennzeichnet, daß** die Laserlichtquelle ein gepulster Ho:YAG-Laser mit einer Wellenlänge im Bereich von 2120 nm ist.

9. Vorrichtung nach einem der Ansprüche 1 bis 7,
**dadurch gekennzeichnet, daß** die Laserlichtquelle ein gepulster Tm:YAG-Laser mit einer Wellenlänge im Bereich von 2010 nm ist.

10. Vorrichtung nach einem der Ansprüche 1 bis 7,
**dadurch gekennzeichnet, daß** die Laserlichtquelle ein gepulster Nd:YAG-Laser mit einer Wellenlänge im Bereich von 1064 bis 1440 nm ist.

11. Vorrichtung nach einem der Ansprüche 1 bis 7,
**dadurch gekennzeichnet, daß** die Laserlichtquelle ein gepulster Er:YAG-Laser mit einer Wellenlänge im Bereich von 2940 nm ist.

12. Vorrichtung nach einem der Ansprüche 1 bis 11,
**dadurch gekennzeichnet, daß** die Laserlichtquelle eine Energie im Bereich von 1 bis 5 J, vorzugsweise 2 J, pro Impuls und eine Impulswiederholfrequenz im Bereich von 2 bis 10 Hz, vorzugsweise von 5 Hz aufweist.

## Claims

1. Apparatus for the laser ablation of tissue, comprising a laser light source (10) whose output power is fed into a first end of an optical fibre (1), wherein the light energy issuing at the free end of the optical fibre (1) is directed on to the tissue (11) to cause ablation of the tissue, wherein the free end of the optical fibre is surrounded at a spacing by a substantially cylindrical sleeve (3) which can be interstitially introduced into the tissue (11) and which comprises a material which is substantially transmissive for the laser light, and wherein beam deflection devices (2, 3) are arranged at the free end of the optical fibre (1) to deflect the light beam issuing from the optical fibre, **characterised in that** the free end of the optical fibre is formed by a conical tip (2) with a tip angle, that the beam deflection devices (2, 3) are formed by the conical tip (2) having the tip angle, the sleeve (3) and the medium filling the intermediate space between the tip (2) and the sleeve (3), and that the beam deflection device (2, 3) provides for coupling out the light energy in a disc-shaped or ring-shaped space (5) at an angle to the longitudinal axis.

2. Apparatus according to claim 1 **characterised in that** the angle to the longitudinal axis is in the range of about 40 to 140°.

3. Apparatus according to claim 1 or claim 2 **characterised in that** the angle to the longitudinal axis is about 90°.

4. Apparatus according to one of claims 1 to 3 **characterised in that** at its free end the sleeve has a tip (6) which facilitates introduction thereof into the tissue.

5. Apparatus according to one of claims 1 to 4 **characterised in that** the tip angle of the tip (2) is in the region of 85°.

6. Apparatus according to one of claims 1 to 5 **characterised in that** the radiation which is discharged into the ring-shaped or disc-shaped space (5) has a divergence angle in the region of 20°.

7. Apparatus according to one of claims 1 to 6 **characterised in that** the outside diameter of the sleeve is in the range of 0.5 to 3 mm, preferably of 2 mm.

8. Apparatus according to one of claims 1 to 7 **characterised in that** the laser light source is a pulsed Ho:YAG laser with a wavelength in the region of 2120 nm.

9. Apparatus according to one of claims 1 to 7 **characterised in that** the laser light source is a pulsed Tm:YAG laser with a wavelength in the region of 2010 nm.

10. Apparatus according to one of claims 1 to 7 **characterised in that** the laser light source is a pulsed Nd:YAG laser with a wavelength in the region of 1064 to 1440 nm.

11. Apparatus according to one of claims 1 to 7 **characterised in that** the laser light source is a pulsed Er:YAG laser with a wavelength in the region of 2940 nm.

12. Apparatus according to one of claims 1 to 11 **characterised in that** the laser light source has an energy in the range of 1 to 5 J, preferably 2 J, per pulse and a pulse repetition rate in the range of 2 to 10 Hz, preferably 5 Hz.

## Revendications

1. Dispositif pour l'ablation laser de tissu, avec une source de lumière laser (10), dont la puissance de sortie est introduite dans une première extrémité d'une fibre optique (1), où l'énergie lumineuse sortant à l'extrémité libre de la fibre optique (1) est dirigée sur le tissu (11) pour provoquer l'ablation du tissu, où l'extrémité libre de la fibre optique est entourée à distance par une gaine (3) sensiblement cylindrique qui peut être introduite de manière interstitielle dans le tissu (11), qui consiste en un matériau sensiblement perméable à la lumière laser, et où des dispositifs de déviation de faisceau (2, 3) pour dévier le faisceau lumineux sortant de la fibre optique sont disposés à l'extrémité libre de la fibre optique (1), **caractérisé en ce que** l'extrémité libre de la fibre optique est formée par une pointe conique (2) ayant un angle de pointe, **en ce que** les dispositifs de déviation de faisceau (2, 3) sont formés par la pointe conique (2) comportant l'angle de pointe, la gaine (3) et le milieu remplissant l'espace intermédiaire entre la pointe (2) et la gaine (3), et **en ce que** le dispositif de déviation de faisceau (2, 3) produit un découplage de l'énergie lumineuse dans un espace (5) en forme de disque ou annulaire sous un angle avec l'axe longitudinal.

2. Dispositif selon la revendication 1, **caractérisé en ce que** l'angle avec l'axe longitudinal est situé dans le domaine d'environ 40 à 140°.

3. Dispositif selon la revendication 1 ou 2, **caractérisé en ce que** l'angle avec l'axe longitudinal est d'environ 90°.

4. Dispositif selon l'une des revendications 1 à 3, **caractérisé en ce que** la gaine comporte à son extrémité libre une pointe (6) qui facilite l'introduction dans le tissu.

5. Dispositif selon l'une des revendications 1 à 4, **caractérisé en ce que** l'angle de pointe de la pointe (2) est situé dans le domaine de 85°.

6. Dispositif selon l'une des revendications 1 à 5, **caractérisé en ce que** le rayonnement délivré dans l'espace (5) annulaire ou en forme de disque présente un angle de divergence dans le domaine de 20°.

7. Dispositif selon l'une des revendications 1 à 16, **caractérisé en ce que** le diamètre externe de la gaine est situé dans le domaine de 0,5 à 3 mm, de préférence à 2 mm.

8. Dispositif selon l'une des revendications 1 à 7, **caractérisé en ce que** la source de lumière laser est un laser Ho:YAG pulsé ayant une longueur d'onde dans le domaine de 2120 nm.

9. Dispositif selon l'une des revendications 1 à 7, **caractérisé en ce que** la source de lumière laser est un laser Tm:YAG pulsé ayant une longueur d'onde dans le domaine de 2010 nm.

10. Dispositif selon l'une des revendications 1 à 7, **caractérisé en ce que** la source de lumière laser est un laser Nd:YAG pulsé ayant une longueur d'onde dans le domaine de 1064 à 1440 nm.

11. Dispositif selon l'une des revendications 1 à 7, **caractérisé en ce que** la source de lumière laser est un laser Er:YAG pulsé ayant une longueur d'onde dans le domaine de 2940 nm.

12. Dispositif selon l'une des revendications 1 à 11, **caractérisé en ce que** la source de lumière laser présente une énergie dans le domaine de 1 à 5 J, de préférence de 2 J, par impulsion et une fréquence de répétition d'impulsions dans le domaine de 2 à 10 Hz, de préférence de 5 Hz.
